(19) Europäisches Patentamt
European Patent Office
Office européen des brevets

(11) Veröffentlichungsnummer: **0 206 101**
**A1**

# (12) EUROPÄISCHE PATENTANMELDUNG

(21) Anmeldenummer: **86107908.5**

(22) Anmeldetag: **10.06.86**

(51) Int. Cl.⁴: **C 07 D 498/06**, C 07 D 471/06, C 07 D 215/56, C 07 C 101/34, C 07 C 121/76, A 61 K 31/535, A 61 K 31/495

(30) Priorität: **22.06.85 DE 3522406**

(43) Veröffentlichungstag der Anmeldung: **30.12.86 Patentblatt 86/52**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**
Erfinder: **Schriewer, Michael, Dr., Am Thelen Siefen 1a, D-5068 Odenthal (DE)**

(54) **Verfahren zur Herstellung von 1,8-verbrückten 4-Chinolon-3-carbonsäuren.**

(57) Die Erfindung betrifft ein Verfahren zur Herstellung von 1,8-verbrückten 4-Chinolon-3-carbonsäuren, deren Zwischenprodukte sowie ihre Verwendung als Arzneimittel, insbesondere als antibakterielle Mittel in der Human- und Veterinärmedizin.

EP 0 206 101 A1

0206101

BAYER AKTIENGESELLSCHAFT 5090 Leverkusen, Bayerwerk
Konzernverwaltung RP
Patentabteilung Ad/Kü-c

Verfahren zur Herstellung von 1,8-verbrückten 4-Chinolon-3-carbonsäuren

Die Erfindung betrifft ein Verfahren zur Herstellung von 1,8-verbrückten 4-Chinolon-3-carbonsäuren, deren Zwischenprodukte sowie ihre Verwendung als Arzneimittel, insbesondere als antibakterielle Mittel in der Human- und Veterinärmedizin.

In der europäischen Patentschrift 0 004 279 wird die Herstellung von 4-Pyridon-3-carbonsäuren durch Umsetzung von tautomeriefähigen Enaminen mit o-Halogen-aryl-carbonsäurehalogeniden und anschließende Cyclisierung in Gegenwart einer Base beschrieben.

Gegenstand der Erfindung ist ein Verfahren zur Herstellung von 1,8-verbrückten 4-Chinolon-3-carbonsäuren und Derivate der Formel (I),

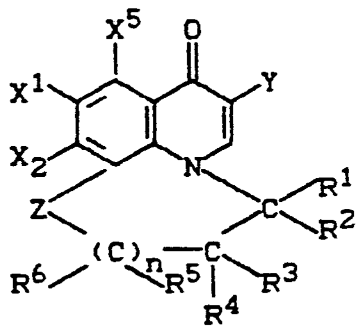

(I)

in der

Y eine Carboxylgruppe, eine Nitrilgruppe, eine Estergruppe-$COOR^7$ oder eine Säureamidgruppe-$CONR^8R^9$ darstellt, wobei $R^7$ für $C_1$-$C_4$-Alkyl steht, $R^8$ und $R^9$ für Wasserstoff oder $C_1$- $C_4$-Alkyl stehen, $R^9$ außerdem gegebenenfalls substituiertes Phenyl sein kann,

$X^1$ für Wasserstoff, Nitro, Alkyl mit 1-3 Kohlenstoffatomen, Halogen, bevorzugt Fluor steht,

$X^2$

Halogen, bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff, einen Aminrest $NR^{10}$ steht, wobei $R^{10}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, eine Alkylgruppe mit 1-3 Kohlenstoffatomen, eine Akoxy- bzw. Alkylmercaptogruppe mit jeweils 1-3 Kohlenstoffatomen substituierten Phenylrest bedeutet, sowie ferner einen Acylrest $R^{11}CO-$ bzw. $R^{12}SO_2-$ darstellt, wobei $R^{11}$ bzw. $R^{12}$ Alkylreste mit 1-6 Kohlenstoffatomen oder gegebenenfalls substituiertes Phenylreste darstellen, sowie ein

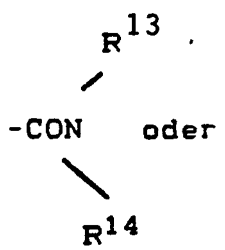 oder 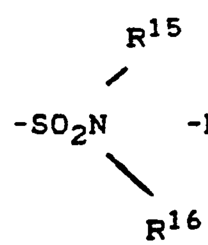 -Rest

sein kann, wobei die Reste $R^{13}$ bis $R^{16}$ Wasserstoff, Alkyl mit 1-6 Kohlenstoffatomen oder einen gegebenenfalls substituierten Phenylrest darstellen,

$R^1$, $R^2$, $R^3$, $R^4$, $R^5$ und $R^6$ für Wasserstoff, eine gegebenenfalls ein- oder mehrfach durch Halogen, insbesondere Chlor oder Fluor, ferner Nitro, Cyano, Hydroxy, Alkoxy bzw. Alkylmercapto mit 1-3 Kohlenstoffatomen, Aryloxy, Arylthio oder eine Estergruppe mit 1-3 Kohlenstoffatomen im Alkoholteil substituierte Alkylgruppe mit 1-6 Kohlenstoffatomen, einen

gegebenenfalls durch Halogen, Nitro, Alkyl bzw. Akoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen, sowie

$R^1$ und $R^2$, $R^3$ und $R^4$, $R^5$ und $R^6$ jeweils mit dem Kohlenstoffatom, das sie verbindet, einen 3-7-gliedrigen Ring bilden, der gegebenenfalls durch evtl. substituierte Alkylreste mit 1-3 Kohlenstoffatomen oder gegebenenfalls substituierte Arylreste substituiert sein kann, und ferner

$R^4$, $R^2$ mit $R^3$ und/oder mit $R^5$ jeweils mit dem Kohlenstoffatomen, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substiutiert ist und

n=0 oder 1 bedeutet und wenn

n=1, $R^5$ und $R^6$ Halogen, Hydroxy, Alkoxy bzw. Alkylmercapto mit 1-3 Kohlenstoffatomen, eine Dialkylaminogruppe mit 1-3 Kohlenstoffatomen in den Alkylresten bedeuten, dadurch gekennzeichnet, daß man Enamine der Formel (II)

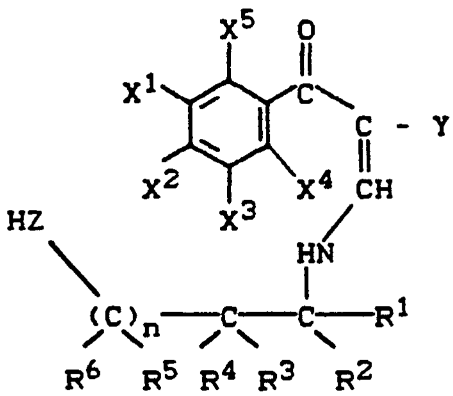

(II)

in der die Reste $X^1$, $X^2$, $X^5$, $R^1$-$R^6$, Z und Y sowie das Symbol n die oben angegebene Bedeutung haben sowie

$X^3$ für Halogen, bevorzugt Fluor oder Chlor sowie die Nitrogruppe steht,

$X^4$ Halogen, bevorzugt Fluor oder Chlor, eine Nitrogruppe, eine Alkoxygruppe mit 1-3 Kohlenstoffatomen im Akylteil, eine Alkylmercapto- bzw. Alkylsulfonylgruppe mit jeweils 1-3 Kohlenstoffatomen im Alkylrest sowie eine Arylsulfonylgruppe bedeutet,

in einer ersten Reaktionsstufe in einem wasserfreien, aprotischen Lösungsmittel in Gegenwart von 1 Äquivalent einer Base zu den 4-Chinolon-3-carbonsäurederivaten der Formel (III) (Methode A),

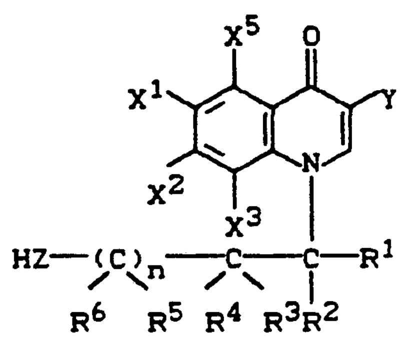

(III)

in der die Reste $X^1$, $X^2$, $X^3$, $X^5$, $R^1$-$R^6$, Z und Y sowie das Symbol n die oben angegebene Bedeutung haben, umsetzt und in einer zweiten Reaktionsstufe in Gegenwart eines weiteren Äquivalents einer Base die Zweitcyclisierung zu den 1,8-verbrückten 4-Chinolon-3-carbonsäurederivaten der oben angegebenen Formel (I) vornimmt, sowie gegebenenfalls die Gruppe Y in an sich bekannter Weise in die Carboxylgruppe und gegebenenfalls diese in ihre Salze überführt.

Der Begriff "substituiertes Aryl" steht für substituiertes Phenyl und substituiertes Naphthyl, bevorzugt für substituiertes Phenyl.

Der Begriff "substituiertes Phenyl" steht für Phenylrest mit bis zu drei Substituenten. Die Substituenten umfassen Alkyl mit 1 - 3 Kohlenstoffatomen, bevorzugt Methyl; Halogen, bevorzugt Chlor und Fluor; Alkoxy mit 1 - 3 Kohlenstoffatomen, bevorzugt Methoxy; Alkylmercapto mit 1 - 3 Kohlenstoffatomen, bevorzugt Methylmercapto; Nitro, Cyano und Estergruppen mit bis zu 3 Kohlenstoffatomen im Alkoholteil.

Besonders vorteilhaft ist die Umsetzung der Enamine (II) mit 2 Äquivalenten einer Base, ohne Zwischenisolierung von (III), in einer sogenannten Eintopf-Reaktion zu den 1,8-verbrückten 4-Chinolon-3-carbonsäurederivaten (I) (Methode B).

Es ist als ausgesprochen überraschend zu bezeichnen, daß die 1,8-Überbrückung in der zweiten Reaktionsstufe des erfindungsgemäßen Verfahrens unter Beteiligung einer durch die meta-ständige Carbonylgruppe mesomer nicht aktivierten Abgangsgruppen $X^3$ erfolgt.

Verwendet man nach Methode A den Enaminoester (<u>1</u>) und Natriumfluorid als Base, so kann der Reaktionsablauf für die 1. Stufe durch das folgende Formelschema wiedergegeben werden:

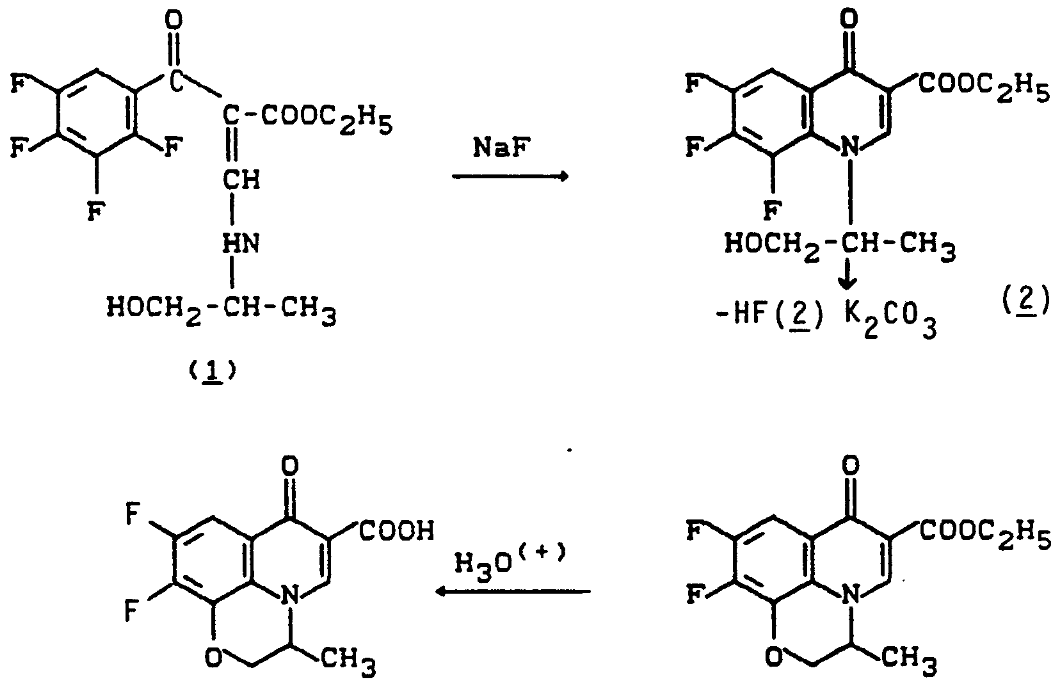


(1) (2)

(3') (3)

Die Zweitcyclisierung von (2) zu (3) wird mit Kaliumcarbonat vorgenommen.

Verwendet man beispielsweise bei der Umsetzung nach Methode B den Enaminoester (4) und Kaliumcarbonat, so kann der Reaktionsablauf durch das folgende Formelschema wiedergegeben werden:

0206101

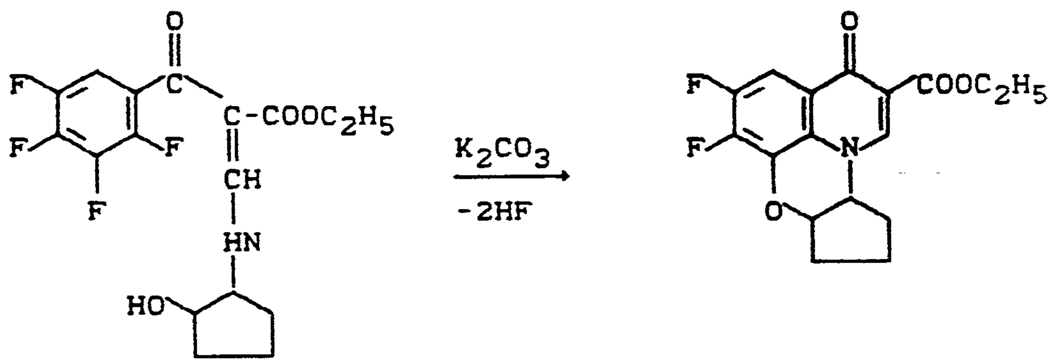


(4) (5)

Die als Ausgangsstoffe nach den Methoden A und B verwendeten Enamine (II) können durch Umsetzung der entsprechenden Enolether (IV) mit den primären Aminen (V) hergestellt werden, wobei $X^1$-$X^5$, $R^1$-$R^6$, Z, Y und n die oben angegebene Bedeutung haben.

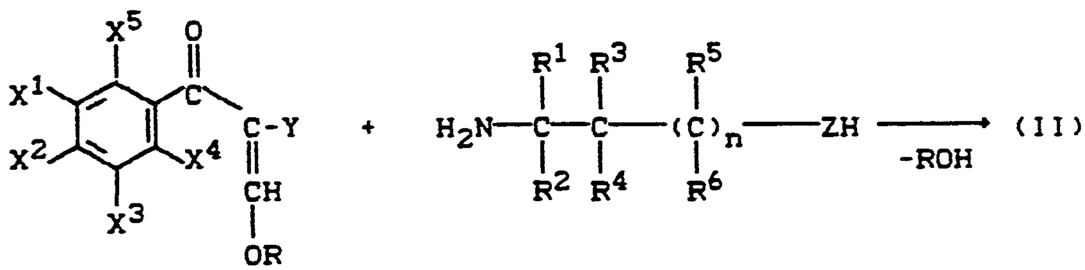


(IV) (V)

R = $CH_3$, $C_2H_5$, $C_3H_{7-n}$

Die Enolether (IV) sind bekannt oder können gemäß folgendem allgemeinen Reaktionsschema hergestellt werden:

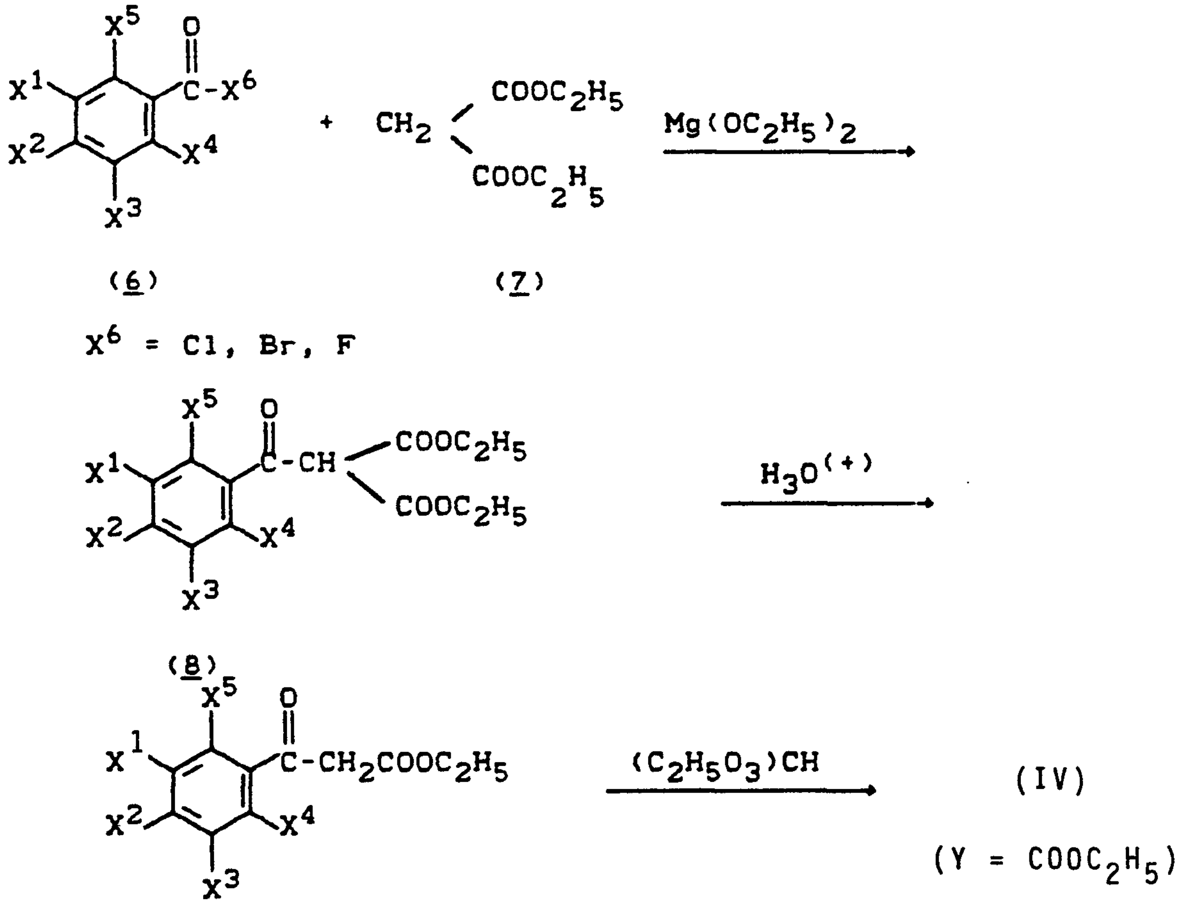


(6) (7)

$X^6$ = Cl, Br, F

(8)

(9)

(IV)

($Y = COOC_2H_5$)

Danach wird Malonsäurediethylester (7) in Gegenwart von Magnesiumethylat mit dem entsprechenden Benzoylhalogenid (6) zum Acylmalonester (8) acyliert (Organicum, 3. Aufl. 1964, S. 438).

Durch partielle Verseifung und Decarboxylierung von (8) in wäßrigem Medium mit katalytischen Mengen Schwefelsäure oder 4-Toluolsulfonsäure erhält man in guter Ausbeute den Benzoylessigsäureethylester (9), der mit Orthoameisensäure- triethylester/Acetanhydrid in den 2-Benzoyl-3-ethoxy- acrylsäureethylester (IV, R = $C_2H_5$) übergeht. Die Umsetzung von (IV) mit den Aminen (V) in einem Lösungsmittel wie z.B. Methylenchlorid, einem Alkohol, Chloroform, Cyclohexan oder Toluol führt in leicht exothermer Reaktion zu den gewünschten Zwischenprodukten (II).

Die Cyclierungsreaktionen (II) $\longrightarrow$ (III), (III) $\longrightarrow$ (I) und (II) $\longrightarrow$ (I) werden in einem Temperaturbereich von etwa 60-300°C, bevorzugt 80-180°C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Die Cyclokondensationen können bei Normaldruck, aber auch bei erhöhtem Druck durchgeführt werden. Im allgemeinen arbeitet man bei Drucken zwischen etwa 1 und etwa 100 bar, vorzugsweise 1 und 10 bar.

Als Säurebinder kommen für die Cyclisierungsreaktionen (II) ⟶ (III), (III) ⟶ (I) und (II) ⟶ (I) Kalium-tert.-butanolat, 1,4-Diaza-bicyclo [2,2,2]-octan (DABCO), 1,8-Diaza-bicyclo [5,4,0]undec-7-en (DBU), Butyl-lithium, Lithium-phenyl, Phenyl-magnesiumbromid, Natriummethylat, Natriumethylat, Natriumhydrid, Natrium- oder Kaliumcarbonat in Betracht. Besonders bevorzugt werden Kalium- oder Natriumfluorid, wenn Fluorwasserstoff abgespalten werden muß.

Für die Primärcyclisierung (II) ⟶ (III) und die Zweitcyclisierung (III) ⟶ (I) wird im allgemeinen jeweils 1 Äquivalent Base verwendet. Werden beide Cyclisierungsreaktionen in der "Eintopf-Reaktion" (II) ⟶ (I) zusammengefaßt, so müssen 2 Äquivalente der oben angegebenen Basen eingesetzt werden. Es kann vorteilhaft sein, bei den Cyclokondensationen (III) ⟶ (I) und (II) ⟶ (I) einen Überschuß von 10 Mol-% Base einzusetzen.

Die im letzten Schritt erfolgende Hydrolyse der Ester, Nitrile und Amide (I) zu den entsprechenden Carbonsäuren kann unter den üblichen bekannten sauren oder basischen Bedingungen erfolgen.

Die als Ausgangsstoffe für diesen Syntheseweg verwendeten 2,3,4,5-Tetrafluorbenzoylchlorid und Pentafluorbenzoylchlorid sind bekannt.

3,5-Dichlor-2,4-difluor-benzoylfluorid (Siedepunkt 97°/20 mbar; $n_D^{20}$ = 1,5148) und 5-Chlor-2,3,4-trifluorbenzoylfluorid (Siedepunkt 66-70°/20 mbar; $n_D^{20}$ = 1,4764) erhält man nebeneinander beim Erhitzen von Tetrachlorbenzoylchlorid mit Kaliumfluorid in Sulfolan auf erhöhte Temperaturen:

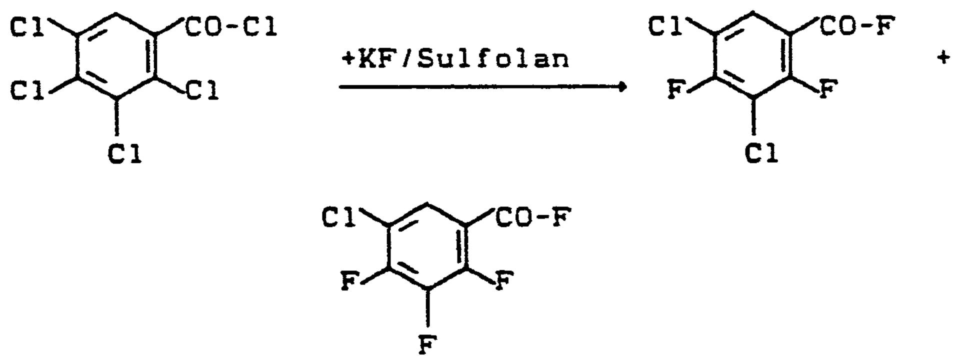


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt: 94°/18 mbar; $n_D^{20}$ = 1,5164) umgesetzt wird:

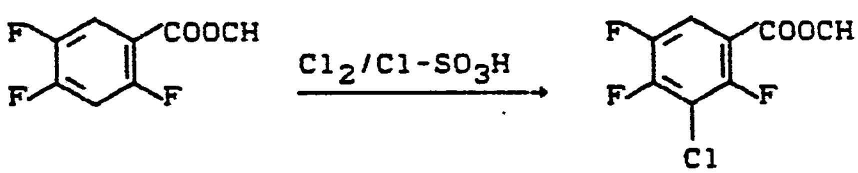

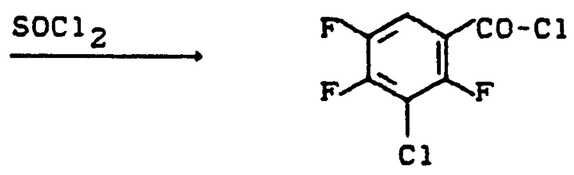


Das 2,4-Dichlor-5-fluor-3-nitro-benzoylchlorid wird durch Nitrierung der bekannten 2,4-Dichlor-5-fluor-benzoesäure zur 2,4-Dichlor-5-fluor-3-nitro-benzoesäure und deren Umsetzung mit Thionylchlorid erhalten.

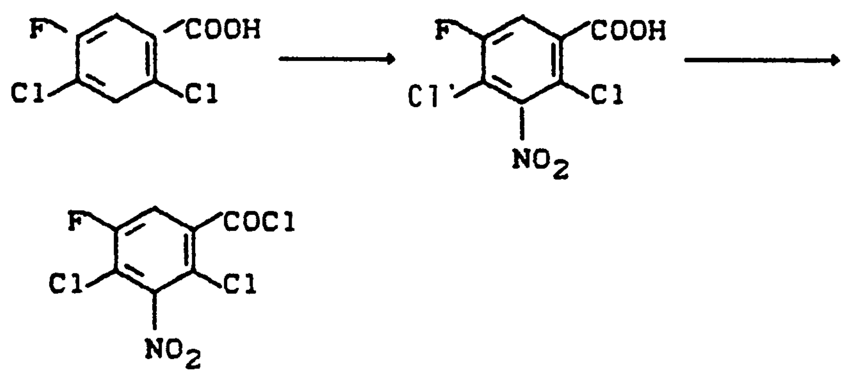

Die als Ausgangsstoffe verwendeten Amine der Formel (V) sind bekannt. Als Beispiele seien genannt:

2-Aminoethanol, 2-Aminopropanol, 3-Aminopropanol, 2-Amino-2-methylpropanol, 2-Aminocyclopentanol, 2-Amino-cyclohexanol, 2-(2-Aminoethylamino)-ethanol, 2-Amino-2-phenylethanol, Ethylendiamin, 2-Aminobutanol, 1,3-Diaminopropan, 1-Amino-2,3-propandiol, 2-Amino-3-phenylpropanol, 2-Amino-1-phenyl-1,3-propandiol, N-Phenyl-ethylen-diamin, N-Benzyl-ethylendiamin, 2-Aminomethyl-cyclohexanol.

Weiterer Gegenstand der Erfindung sind 1,8-verbrückte 4-Chinolon-3-carbonsäurederivate der Formel (I')

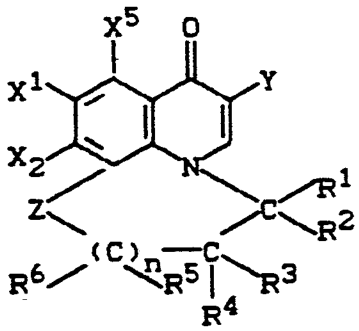

(I')

in der

$X^1$, $X^2$, $X^5$, $R^1$ bis $R^6$, Z und Y sowie n die oben angegebene Bedeutung haben, mit der Maßgabe, daß $X^1$ und $X^2$ nicht beide für Fluor stehen können, wenn n gleich 0 ist und $R^1$ und $R^4$ für Wasserstoff und $R^2$ und $R^3$ für Wasserstoff oder niedrig Alkyl stehen.

Ebenso gehören zum Gegenstand der Erfindung die Verbindungen der Formeln (II) und (III).

Die erfindungsgemäßen 1,8-verbrückten 4-Chinolon-3-carbonsäuren der Formeln (I) und (I') und ihre Derivate sind antibakteriall wirksam. Sie können ferner als Zwischenprodukte zur Synthese von hochwirksamen Bakteriziden dienen. So erhält man durch Umsetzung der Pyrido-benzoxaZincarbonsäure (3') mit 1-Methylpiperazin das Bakterizid Ofloxacin.

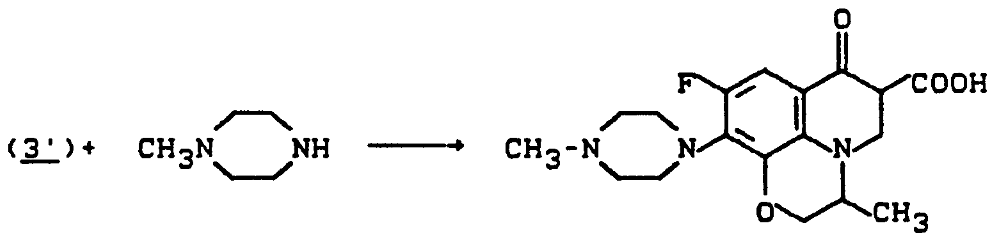

Ofloxacin

Die folgenden Beispiele erläutern die Erfindung:

Herstellung der Ausgangsverbindungen

Beispiel A

2-(2,4-Dichlor-5-fluor-3-nitro-benzoyl)-3-ethoxy-acrylsäureethylester

a) 2,4-Dichlor-5-fluor-3-nitro-benzoesäure

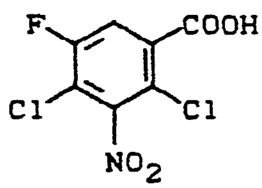

Unter Eiskühlung und Rühren werden 34 ml konz. Schwefelsäure tropfenweise mit 40 ml konz. Salpetersäure versetzt. In dieses Nitriergemisch trägt man portionsweise 20,9 g 2,4-Dichlor-5-fluorbenzoesäure ein, wobei die Temperatur auf 45-50°C steigt. Dann wird noch 3 Stunden auf 90-100°C erhitzt, das auf Raumtemperatur abgekühlte Gemisch auf 350 ml Eiswasser gegossen, der Niederschlag abgesaugt und mit Wasser gewaschen. Das feuchte Rohprodukt wurde in 30 ml Methanol heiß gelöst und die Lösung mit 150 ml $H_2O$ versetzt. Der Niederschlag wird kalt abgesaugt, mit $CH_3OH/H_2O$ gewaschen und im Vakuum bei 80°C getrocknet. Es werden 21,2 g rohe 2,4-Dichlor-5-fluor-3-nitro-benzoesäure erhalten. Sie ist genügend rein für die weiteren Umsetzungen. Eine Probe aus Toluol/Petrolether umkristallisiert liefert Kristalle vom Schmelzpunkt 192°C.

0206101

b) 2,4-Dichlor-5-fluor-3-nitro-benzoylchlorid

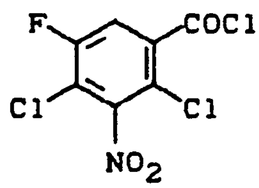

106,6 g 2,4-Dichlor-5-fluor-3-nitro-benzoesäure werden mit 250 ml Thionylchlorid 2 Stunden unter Rückfluß zum Sieden erhitzt. Das überschüssige Thionylchlorid wird dann bei Normaldruck abdestiliert und der Rückstand im Feinvakuum fraktioniert. Bei 110-115°C/0,08-0,09 mbar gehen 104,7 g 2,4-Dichlor-5-fluor-3-nitro-benzoylchlorid über. Beim Stehen bilden sich Kristalle vom Schmelzpunkt 35-37°C.

c) (2,4-Dichlor-5-fluor-3-nitro-benzoyl)-essigsäureethylester

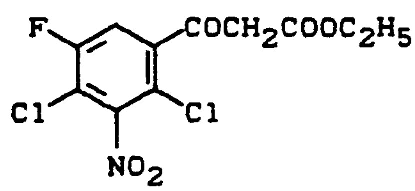

10,1 g Magnesiumspäne werden in 21 ml Ethanol mit 2,1 g Tetrachlormethan versetzt und nach Beginn der Wasserstoff-Entwicklung ein Gemisch aus 66,6 g Malonsäurediethylester, 40 ml Ethanol und 150 ml

Toluol bei 50-60°C tropfenweise zugefügt. Man rührt 1 Stunden bei dieser Temperatur nach, kühlt auf -5 bis -10°C und tropft langsam eine Lösung von 109,2 g 2,4-Dichlor-5-fluor-3-nitro-benzoylchorid in 50 ml Toluol zu. Danach wird 1 Stunde bei 0°C gerührt, über Nacht auf Raumtemperatur gebracht und noch 2 Stunden auf 40-50°C erwärmt.Das Reaktionsgemisch wird unter Eiskühlung mit einem Gemisch aus 160 ml Wasser und 10,4 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen mit Natriumsulfat getrocknet und das Lösungsmittel abgezogen. Man erhält 144,5 g (2,4-Dichlor-5-fluor-3-nitro-benzoyl)-malonsäurediethylester als Rohprodukt. Dieses wird nach Zugabe von 200 ml Wasser und 0,6 g 4-Toluolsulfonsäure 3 Stunden unter Rückfluß erhitzt, die Mischung mit Methylenchlorid extrahiert, der Extrakt mit Natriumsulfat getrocknet und das Lösungsmittel im Vakuum abdestilliert. Es werden 118 g substituierter Benzoylessigester als Rohprodukt erhalten. Er besitzt eine für die weiteren Umsetzungen genügende Reinheit.

d) 2-(2,4-Dichlor-5-fluor-3-nitro-benzoyl)-3-ethoxy-acrylsäure-ethylester

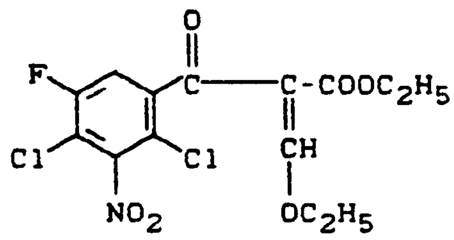

244,8 g (2,4-Dichlor-5-fluor-3-nitro-benzoyl)-essigsäureethylester werden mit 166 g Orthoameisensäuretriethylester und 185 g Essigsäureanhydrid 3 Stunden auf 150-160°C erhitzt. Man engt dann im Vakuum ein und erhält 270 g Benzoyl-ethoxy-acrylsäure-ethylester als öligen Rückstand.

Beispiel 1

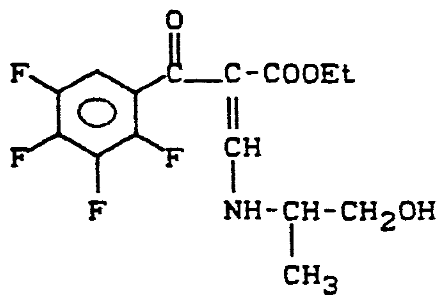

8 g 3-Ethoxy-2-(2,3,4,5-tetrafluorbenzoyl)-acrylsäure-ethylester (Le A 22 302) werden in 10 ml Ethanol vorgelegt. Unter Eiskühlung wird eine Lösung von 2,06 g DL-2-Amino-1-propanol in 10 ml Ethanol zugetropft. Man läßt auf Raumtemperatur erwärmen und rührt zwei Stunden. Danach wird im Vakuum eingeengt. Es bleiben 9,1 g 2-(2,3,4,5-tetrafluorbenzoyl)-3-(1-hydroxy-2-propyl-amino)acrylsäureethylester als rohes Öl zurück.

Analog Beispiel 1 werden folgenden Aminoacrylester erhalten (Tabelle 1).

T a b e l l e   1

Aminoacrylester der Formel (II)
n = 0, Z = 0 (Sauerstoff)

| Beispiel | $X_1$ | $X_2$ | $X_3$ | $X_4$ | $X_5$ | $R_1$ | $R_2$ | $R_3$ | $R_4$ | Y | Schmelzpunkt |
|---|---|---|---|---|---|---|---|---|---|---|---|
| 2* | F | Cl | $NO_2$ | Cl | H | H | H | H | H | COOEt | Öl |
| 3* | F | F | F | H | H | H | H | H | H | " | Öl |
| 4* | F | Cl | $NO_2$ | Cl | H | Me | H | H | H | " | Öl |
| 5 | F | F | F | F | H | H | H | Me | H | " | Öl |
| 6* | F | Cl | $NO_2$ | Cl | H | H | H | Me | H | " | Öl |
| 7 | F | F | F | F | H | Me | Me | H | H | " | 93-94° |
| 8* | F | Cl | $NO_2$ | Cl | H | Me | Me | H | H | " | 78-80° |
| 9 | F | F | F | F | H | Me | Me | Me | H | " | Öl |
| 10 | F | F | F | F | H | $-(CH_2)_5-$ | | H | H | " | Öl |
| 11 | F | F | F | F | H | H | H | Ph | H | " | 117-19° |
| 12* | F | Cl | $NO_2$ | Cl | H | H | H | Ph | H | " | 148-50° |
| 13 | F | F | F | F | H | iPr | H | H | H | " | Öl |
| 14 | F | F | F | F | H | $CH_2OH$ | H | H | H | " | Öl |
| 15 | F | F | F | F | H | H | H | $CH_2OH$ | H | " | Öl |
| 16 | F | F | F | F | H | H | H | $CH_2F$ | H | " | Öl |
| 17 | F | F | F | F | H | Et | H | H | H | " | Öl |
| 18 | F | F | F | F | H | H | $-(CH_2)_3-$ | | H | " | Öl |
| 19 | F | F | F | F | H | H | $-(CH_2)_4-$ | | H | " | Öl |
| 20** | F | Me | $NO_2$ | Cl | H | H | H | H | H | " | Öl |

* hier wird der 2-(2,4-Dichlor-5-fluor-3-nitro-benzoyl)-3-ethoxyacrylsäureester Beispiel A) eingesetzt.

** hier wird der 2-(2-Chlor-5-fluor-4-methyl-3-nitrobenozyl)-3-ethoxyacrylsäureethylester eingesetzt.

| Beispiel | $X_1$ | $X_2$ | $X_3$ | $X_4$ | $X_5$ | $R_1$ | $R_2$ | $R_3$ | $R_4$ | $R_5$ | $R_6$ | Y | Schmelzpunkt |
|---|---|---|---|---|---|---|---|---|---|---|---|---|---|
| 21 | F | F | F | F | H | H | H | H | H | H | H | COOEt | 77-79° |
| 22 | F | F | F | F | H | H | $-(CH_2)_4-$ | | H | H | H | " | Öl |
| 23* | F | Cl | $NO_2$ | Cl | H | H | H | H | H | H | H | " | Öl |

* hier wird der 2-(2,4-Dichlor-5-fluor-3-nitro-benzoyl)-3-ethoxyacrylsäureester Beispiel A) eingesetzt.

** hier wird der 2-(2-Chlor-5-fluor-4-methyl-3-nitrobenzoyl)-3-ethoxyacrylsäure-ethylester (DE-OS 3 441 788) eingesetzt.

Stufenweise Cyclisierung von Aminoacrylestern (Methode A)

Beispiel 24

a)

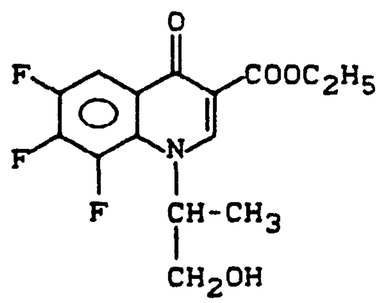

9 g Produkt aus Beispiel 1 werden mit 3,2 g NaF in 66 ml DMSO vier Stunden auf 140°C erhitzt. Nach Abkühlung auf Raumtemperatur wird mit Wasser verdünnt und der Niederschlag isoliert. Ausbeute: 7,6 g 6,7,8-Trifluor-1,4-dihydro-1-(1-hydroxy-2-propyl)-4-oxo-3-chinolincarbonsäureethylester. Schmelzpunkt: 155-57°C.

b)

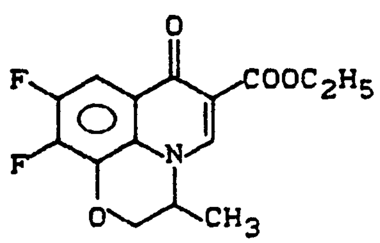

7,6 g der Verbindung aus a) werden mit 38 g Kaliumcarbonat in 34 ml DMF zwei Stunden auf 140-45°C erhitzt. Nach Abkühlen auf Raumtemperatur und verdünnen mit Wasser wird der entstandene Feststoff isoliert. Ausbeute: 4,8 g 9,10-Difluor-2,3-dihydro-3-methyl-7-oxo-7H-pyrido[1,2,3-de] [1,4]-benzoxazin-6-carbonsäureethylester. Schmelzpunkt 240-2°C.

Einstufige Cyclisierung (Methode B)

Beispiel 25

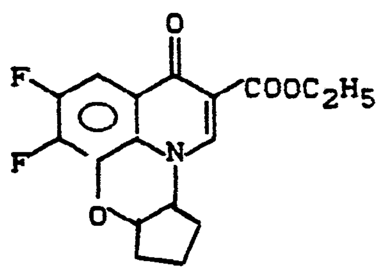

8 g des Produkts aus Beispiel 18 werden in 40 ml DMF mit 3,3 g Kaliumcarbonat vier Stunden auf 140-45°C erhitzt. Nach Abkühlen auf Raumtemperatur wird mit Wasser verdünnt und gekühlt. Den enstandenen Niederschlag trennt man ab und kristallisiert ihn gegebenenfalls aus Glykolmonomethylether um. Ausbeute: 5,4 g 5,6-Difluor-3a, 11a-dihydro-8-oxo-8H-cyclopenta [1,2-b] pyrido [1,2,3-de] [1,4]-benzoxazin-9-carbonsäureethylester. Schmelzpunkt: 255-58°.

Analog Beispiel 25 werden folgende Chinoloncarbonsäureester erhalten (Tabelle 2):

T a b e l l e  2

Chinoloncarbonsäureester der Formel (I)
n = 0, Z = 0 (Sauerstoff)

| Beispiel | $X_1$ | $X_2$ | $X_5$ | $R_1$ | $R_2$ | $R_3$ | $R_4$ | $R_5$ | $R_6$ | Y | Schmelzpunkt |
|---|---|---|---|---|---|---|---|---|---|---|---|
| 26 | F | Cl | H | H | H | H | H | | | COOEt | 225-60° |
| 27 | F | F | H | H | H | H | H | | | COOEt | 229-32° |
| 28 | F | Cl | H | Me | H | H | H | | | COOEt | 250° |
| 29 | F | F | H | H | H | Me | H | | | COOEt | 248-50° |
| 30 | F | Cl | H | H | H | Me | H | | | COOEt | 256-8° |
| 31 | F | F | H | Me | Me | H | H | | | COOEt | 288-90° |
| 32 | F | F | H | Me | H | Me | H | | | COOEt | 186-9° |
| 33 | F | F | H | $-(CH_2)_5-$ | | H | H | | | COOEt | 234-40° |
| 34 | F | F | H | H | H | Ph | H | | | COOEt | 274-6° |
| 35 | F | Cl | H | H | H | Ph | H | | | COOEt | 280-2° |
| 36 | F | F | H | iPr | H | H | H | | | COOEt | 105-10° |
| 37 | F | F | H | $CH_2OH$ | H | H | H | | | COOEt | 253-55° |
| 38 | F | F | H | H | H | $CH_2OH$ | H | | | COOEt | 228-30° |
| 39 | F | F | H | H | H | $CH_2F$ | H | | | COOEt | 226-8° |
| 40 | F | F | H | Et | H | H | H | | | COOEt | 226-8° |
| 41 | F | F | H | H | $-(CH_2)_4-$ | | H | | | COOEt | 278-9° |
| 42 | F | Me | H | H | H | H | H | | | COOEt | > 300° |
| 43 | F | F | H | H | H | H | H | H | H | COOEt | 212-15° |
| 44 | F | F | H | H | $-(CH_2)_4-$ | | H | H | H | COOEt | 178-82° |
| 45 | F | Cl | H | H | H | H | H | H | H | COOEt | 142-4° |

Beispiel 46

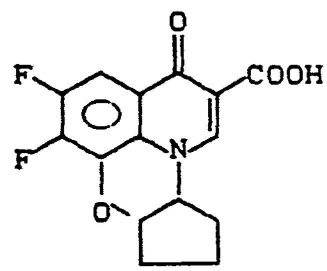

5,4 g des Produktes aus Beipsiel 25 werden in einem Gemisch aus 17 ml Essigsäure, 16 ml Wasser und 1,6 ml konzentrierter Schwefelsäure vier Stunden auf 140°C (Bad) erhitzt. Anschließend wird gekühlt, mit Wasser verdünnt und der Feststoff isoliert.
Ausbeute: 4,4 g
Schmelzpunkt: 270° (Z).

Analog Beispiel 46 werden folgende Chinoloncarbonsäuren erhalten (Tabelle 3):

0206101

<u>T a b e l l e   3</u>

Chinoloncarbonsäuren der Formel (I)
n = O , Z = O (Sauerstoff)

| Beispiel | $X_1$ | $X_2$ | $X_5$ | $R_1$ | $R_2$ | $R_3$ | $R_4$ | $R_5$ | $R_6$ | Y | Schmelzpunkt |
|---|---|---|---|---|---|---|---|---|---|---|---|
| 47 | F | F | H | H | H | H | H | | | COOH | 270-3° |
| 48 | F | F | H | Me | H | H | H | | | COOH | > 300° |
| 49 | F | F | H | H | H | Me | H | | | COOH | 288-90° |
| 50 | F | Cl | H | H | H | Me | H | | | COOH | 299-300° |
| 51 | F | F | H | Me | Me | H | H | | | COOH | > 300° |
| 52 | F | F | H | Me | H | Me | H | | | COOH | 268-70° |
| 53 | F | F | H | $-(CH_2)_5-$ | | H | H | | | COOH | > 300° |
| 54 | F | Cl | H | H | H | Ph | H | | | COOH | > 300° |
| 55 | F | F | H | H | H | Ph | H | | | COOH | 300° (Z) |
| 56 | F | F | H | iPr | H | H | H | | | COOH | 290-94° |
| 57 | F | F | H | $CH_2OH$ | H | H | H | | | COOH | 244-46° |
| 58 | F | F | H | H | H | $CH_2OH$ | H | | | COOH | 148-50° |
| 59 | F | F | H | Et | H | H | H | | | COOH | 292-4° |
| 60 | F | F | H | H | $-(CH_2)_4-$ | | H | | | COOH | 294-6° |
| 61 | F | Me | H | H | H | H | H | | | COOH | > 300° |
| 62 | F | F | H | H | H | H | H | H | H | COOH | 230-3° (Z) |
| 63 | F | F | H | H | $-(CH_2)_4-$ | | H | H | H | COOH | 274-6° |
| 64 | F | Cl | H | H | H | H | H | H | H | COOH | 270° (Z) |

Beispiel 65

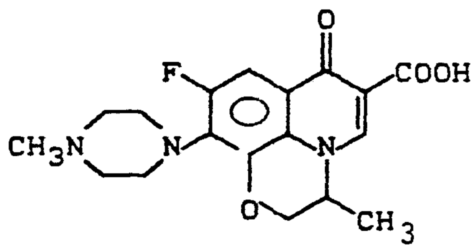

26 g Produkt aus Beispiel 48 und 28,7 g N-Methylpiperazin werden in 290 ml DMSO 2 1/2 Stunden auf 140°C erhitzt. Anschließend destilliert man alle flüchtigen Bestandteile im Hochvakuum ab. Der Rückstand wird mit Ethanol aufgekocht. Man läßt abkühlen und trennt den Feststoff ab.

Ausbeute: 23 g 9-Fluor-2,3-dihydro-3-methyl-10-(1-methylpiperazinyl)-7-oxo-7H-pyrido [1,2,3-de] [1,4]-benzoxazin-6-carbonsäure (Ofloxacin). Schmelzpunkt: 278-80° (Z).

Beispiel 66

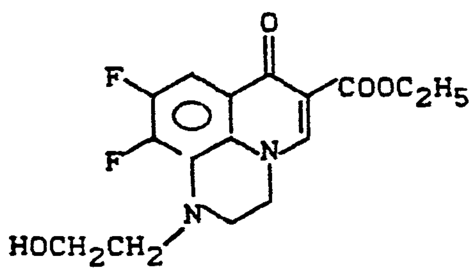

6,4 g 3-Ethoxy-2-(2,3,4,5-tetrafluorbenzoyl)-acrylsäure-ethylester werden in 8 ml Ethanol vorgelegt. Unter Eiskühlung werden nun 2,3 g 2-(2-Aminoethylamino)-ethanol gelöst in 15 ml Ethanol zugetropft. Man läßt auf Raumtemperatur kommen und rührt zwei Stunden. Anschließend wird im Vakuum eingedampft. Der ölige Rückstand besteht aus 8,6 g rohem Aminoacrylester; 8,4 g des rohen Aminoacrylesters werden mit 3,3 g Kaliumcarbonat in 40 ml DMF vier Stunden auf 140°C erhitzt. Nach Abkühlen auf Raumtemperatur wird mit Wasser verdünnt und der Niederschlag isoliert. Nach Trocknen im Vakuum über KOH beträgt die Ausbeute 3,1 g 9,10-Difluor-2,3-dihydro-1-(2-hydroxyethyl)-7-oxo-7H-pyrido [1,2,3-de] chinoxalin-6-carbonsäureethylester.
Schmelzpunkt: 244-5°.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die

folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen im Vergleich zu Ciprofloxacin angegeben.

MHK (mcg/ml)

| Stamm | Beispiel Nr. 61 | Beispiel Nr. 64 |
|---|---|---|
| E. coli Neumann | 0,125 | 0,5 |
| E. coli T 7 | 0,5 | 1 |
| E. coli A 261 | 0,5 | 1 |
| Klebsiella 8085 | 0,125 | 0,25 |

Agar dilutionstest (Isosensitest-Medium); Denley Multipointinoculator

Patentansprüche

1. Verfahren zur Herstellung von 1,8-verbrückten 4-Chinolon-3-carbonsäuren der Formel I

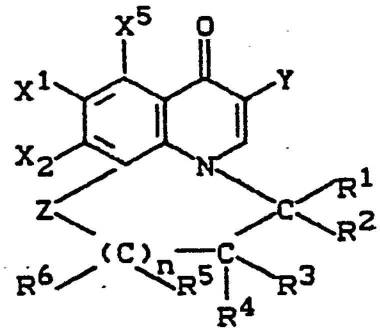

(I)

in der

Y eine Carboxylgruppe, eine Nitrilgruppe, eine Estergruppe-$COOR^7$ oder eine Säureamidgruppe-$CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1-3 Kohlenstoffatomen, Halogen, bevorzugt Fluor steht,

$X^2$ Halogen, bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff, einen Aminrest $NR^{10}$ steht, wobei $R^{10}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, eine Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{11}$ CO- bzw., $R^{12}$ $SO_2$- darstellt, wobei $R^{11}$ bzw. $R^{12}$ Alkylreste mit 1-6 Kohlenstoffatomen oder gegebenenfalls substituierte Phenylreste darstellen, sowie ein

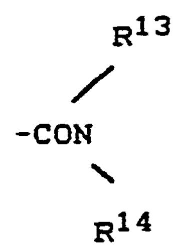 oder 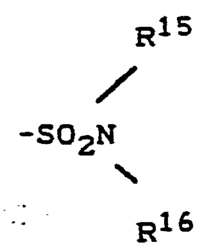 -Rest

sein kann, wobei die Reste $R^{13}$ bis $R^{16}$ Wasserstoff, Alkyl mit 1-6 Kohlenstoffatomen oder einen gegebenenfalls substituierten Phenylrest darstellen,

$R^1$, $R^2$, $R^3$, $R^4$, $R^5$ und $R^6$ für Wasserstoff, eine gegebenenfalls ein- oder mehrfach durch Halogen, insbesondere Chlor oder Fluor, ferner Nitro, Cyano, Hydroxy, Alkoxy bzw. Alkylmercapto mit 1-3 Kohlenstoffatomen, Aryloxy, Arylthio oder eine Estergruppe mit 1-3 Kohlenstoffatomen im Alkoholteil substituierte Alkylgruppe mit 1-6 Kohlenstoffatomen, einen gegebenenfalls durch Halogen, Nitro, Alkyl- bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen, sowie

$R^1$ und $R^2$, $R^3$ und $R^4$, $R^5$ und $R^6$ jeweils mit dem Kohlenstoff, das sie verbindet, einen 3-7 gliedrigen Ring bilden, der gegebenenfalls durch evtl. substituierte Alkylreste mit 1-3 Kohlenstoffatomen oder gegebenenfalls substituierte Arylreste substituiert sein kann, und ferner

$R^2$ mit $R^3$ und/oder $R^4$ mit $R^5$ jeweils mit dem Kohlenstoffatom, an das sie gebunden sind, einen 3-7 gliedrigen Ring bilden, der gegebenenfalls durch gegebenenfalls substituierte Alkylreste mit 1-3 Kohlenstoffatomen oder gegebenenfalls substituierte Arylreste substituiert ist, und

n = 0 oder 1 bedeutet, und wenn n = 1 , $R^5$ und $R^6$ Halogen, Hydroxy, Alkoxy bzw. Alkylmercapto mit 1-3 Kohlenstoffatomen, eine Dialkylaminogruppe mit 1-3 Kohlenstoffatomen in den Alkylresten bedeuten,

dadurch gekennzeichnet, daß man Enamine der Formel I·I

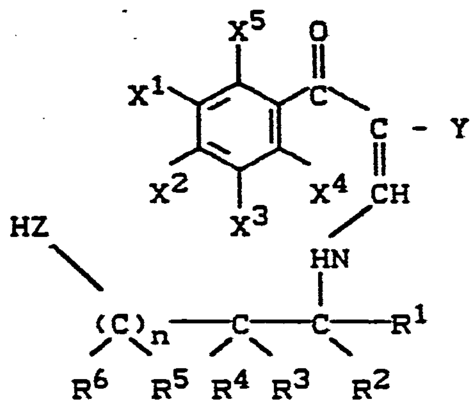

(II)

in der

die Reste $X^1$, $X^2$, $X^3$, $X^4$, $X^5$, $R^1$-$R^6$, Z und Y sowie das Symbol n die oben angegebene Bedeutung haben,

in einer ersten Reaktionsstufe in einem wasserfreien aprotischen Lösungsmittel in Gegenwart von 1 Äquivalent einer Base bei Temperaturen von 80°C bis 180°C zu 4-Chinolon-3-carbonsäurederivaten der Formel III

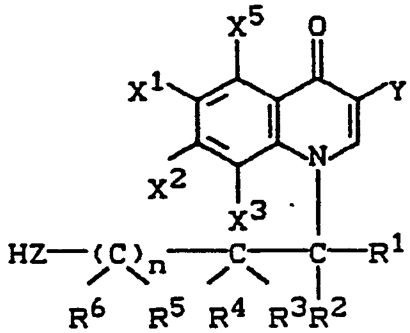

(III)

in der die Reste $X^1$, $X^2$, $X^3$, $X^5$, $R^1$-$R^6$, Z und Y sowie das Symbol n die oben angegebene Bedeutung haben, umsetzt, und in einer zweiten Reaktionsstufe in Gegenwart eines weiteren Äquivalents einer Base die Zweitcyclisierung zu den 1,8-verbrückten 4-Chinolon-3-carbonsäurederivaten der obengenannten Formel I vornimmt, sowie gegebenenfalls die Gruppe Y in an sich bekannter Weise in die Carboxylgruppe und gegebenenfalls diese in ihre Salze überführt.

2. Verfahren nach Anspruch 1, dadurch gekennzeichnet, daß man die Enamine der Formel II mit 2 Äqivalenten einer Base, ohne Zwischenisolierung der Verbindungen III, in einem Eintopfverfahren zu den 1,8-überbrückten 4-Chinolon-3-carbonsäurederivaten I umsetzt.

3. Verfahren nach den Ansprüchen 1 und 2, dadurch gekennzeichnet, daß man die Cyclisierungsreaktionen (II)⟶(III), (III)⟶(I( und (II)⟶(I) bei Temperaturen von 50 bis 300 °C in Gegenwart von Dioxan, DMSO, N-Vinylpyrrolidin, Sulfolan, Hexamethylphosphorsäuretrisamid oder DMF oder in Gegenwart eines Gemisches dieser Lösungsmittel und in Gegenwart eines Säurebinders durchführt.

4. **Enamine der allgemeinen Formel II**

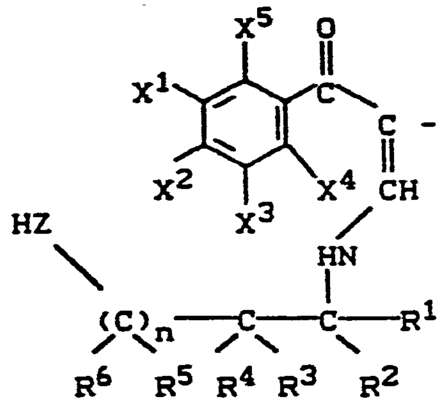

(II)

in der

Y eine Carboxylgruppe, eine Nitrilgruppe, eine Estergruppe-$COOR^7$ oder eine Säureamidgruppe-$CONR^8R^9$ darstellt, wobei $R^7$ für $C_1$-$C_4$-Alkyl steht, $R^8$ und $R^9$ für Wasserstoff oder $C_1$- $C_4$-Alkyl stehen, $R^9$ außerdem gegebenenfalls substituiertes Phenyl sein kann,

$X^1$ für Wasserstoff, Nitro, Alkyl mit 1-3 Kohlenstoffatomen, Halogen, bevorzugt Fluor steht,

$X^2$ Halogen, bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff, einen Aminrest $NR^{10}$ steht, wobei $R^{10}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, eine Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{11}CO$- bzw. $R^{12}SO_2$- darstellt, wobei $R^{11}$ bzw. $R^{12}$ Alkylreste mit 1-6 Kohlenstoffatomen oder gegebenenfalls substituiertes Phenylreste darstellen, sowie ein

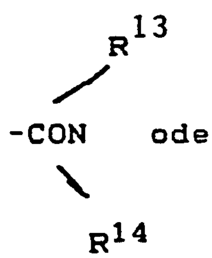 oder 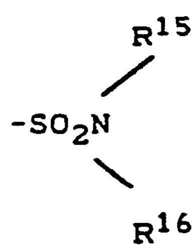 -Rest

sein kann, wobei die Reste $R^{13}$ bis $R^{16}$ Wasserstoff, Alkyl mit 1-6 Kohlenstoffatomen oder einen gegebenenfalls substituierten Phenylrest darstellen,

$R^1$, $R^2$, $R^3$, $R^4$, $R^5$ und $R^6$ für Wasserstoff, eine gegebenenfalls ein- oder mehrfach durch Halogen,

insbesondere Chlor oder Fluor, ferner Nitro, Cyano, Hydroxy, Alkoxy bzw. Alkylmercapto mit 1-3 Kohlenstoffatomen, Aryloxy, Arylthio oder eine Estergruppe mit 1-3 Kohlenstoffatomen im Alkoholteil substituierte Alkylgruppe mit 1-6 Kohlenstoffatomen, einen gegebenenfalls durch Halogen, Nitro, Alkyl bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen, sowie

$R^1$ und $R^2$, $R^3$ und $R^4$, $R^5$ und $R^6$ jeweils mit dem Kohlenstoffatom das sie verbindet einen 3-7 gliedrigen Ring bilden, der gegebenenfalls durch evtl. substituierte Alkylreste mit 1 - 3 Kohlenstoffatomen oder gegebenenfalls substituierte Arylreste substituiert sein kann, und ferner

$R^2$ mit $R^3$ und/oder $R^4$ und $R^5$ jeweils mit den Kohlenstoffatomen,an die sie gebunden sind, einen 3-7 gliedrigen Ring bilden, der durch gegebenenfalls substituierte Alkylreste mit 1-3 Kohlenstoffatomen oder gegebenenfalls substituierte Arylreste substituiert ist, und

n = 0 oder 1 bedeutet und wenn

n = 1, $R^5$ und $R^6$ Halogen, Hydroxy, Alkoxy bzw. Alkylmercapto mit 1-3 Kohlenstoffatomen, eine eine Dialkylaminogruppe mit 1-3 Kohlenstoffatomen in den Alkylresten bedeuten, und

$X^3$ für Halogen, bevorzugt Fluor oder Chlor sowie die Nitrogruppe steht,

$X^4$ Halogen, bevorzugt Fluor oder Chlor, eine Nitrogruppe, eine Alkoxygruppe mit 1-3 Kohlenstoffatomen im Alkylteil, eine Alkylmercapto- bzw. Alkylsulfonylgruppe mit jeweils 1-3 Kohlenstoffatomen im Alkylrest sowie eine Arylsulfonylgruppe bedeutet.

5. 4-Chinolon-3-carbonsäurederivate der Formel III

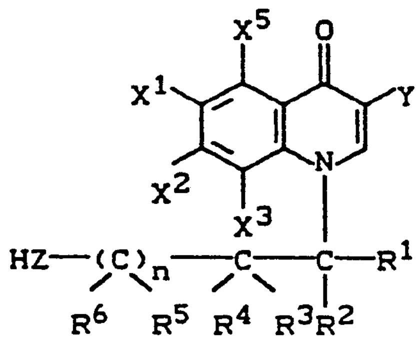

(III)

in der die Reste $X^1$, $X^2$, $X^3$, $X^5$, $R^1$ bis $R^6$, Z und Y sowie das Symbol n die in Anspruch 3 angegebene Bedeutung haben.

6. 1,8-verbrückte 4-Chinolon-3-carbonsäurederivate der Formel I'

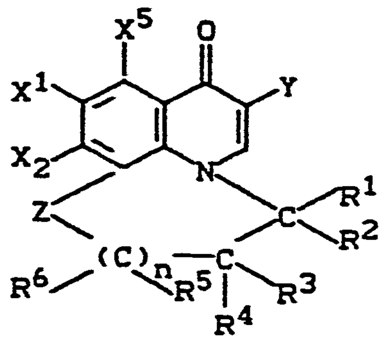

(I')

in der

$X^1$, $X^2$, $X^5$, $R^1$ bis $R^6$, Z und Y sowie n die in Anspruch 3 angegebene Bedeutung haben, mit der Maßgabe, daß $X^1$ und $X^2$ nicht beide für Fluor stehen können, wenn n gleich O ist und $R^1$ und $R^4$ für Wasserstoff und die Reste $R^2$ und $R^3$ für Wasserstoff oder niedrig Alkyl stehen.

7. Verwendung von Verbindungen der Formel I' gemäß Anspruch 6 zur Herstellung von Verbindungen, die zur therapeutischen Anwendung am menschlichen oder tierischen Körper geeignet sind.

8. Arzneimittel, enthaltend Verbindungen der Formel I' gemäß Anspruch 6.

9. Verwendung von Verbindungen der Formel I' gemäß Anspruch 6 zur Herstellung von Arzneimitteln.

10. Verfahren zur Herstellung der Verbindungen der Formel (II) gemäß Anspruch 1 durch Umsetzung der entsprechenden Enolether der Formel (IV)

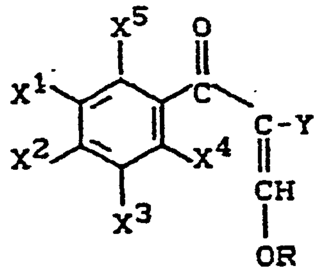

(IV)

mit

$R = CH_3$, $C_2H_5$, $C_3H_7$-n

mit den primären Aminen der Formel (V)

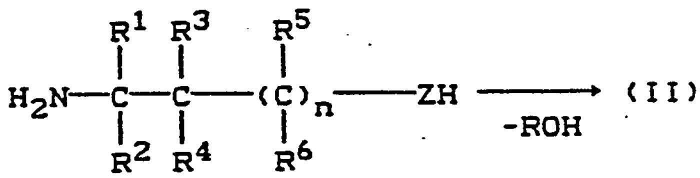


(V)

wobei $X^1$-$X^5$, $R^1$-$R^6$, Z, Y und n die in Anspruch 1 angegebene Bedeutung haben.

Patentansprüche

1. Verfahren zur Herstellung von 1,8-verbrückten 4-Chinolon-3-carbonsäuren der Formel I

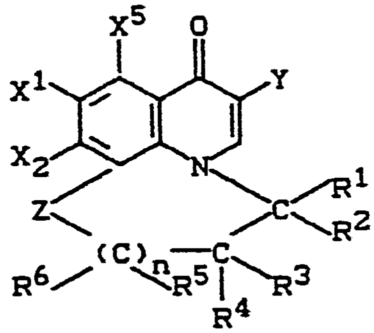

(I)

in der

Y eine Carboxylgruppe, eine Nitrilgruppe, eine Estergruppe-$COOR^7$ oder eine Säureamidgruppe-$CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1-3 Kohlenstoffatomen, Halogen, bevorzugt Fluor steht,

$X^2$ Halogen, bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff, einen Aminrest $NR^{10}$ steht, wobei $R^{10}$ Wasserstoff, einen gegebenenfalls durch Halogen, Trifluormethyl, Nitro, Cyano, Hydroxy, Alkoxy bzw. Alkylmercapto mit 1-3 Kohlenstoffatomen, Aryloxy, Arylthio oder Esterrest mit 1-3 Kohlenstoffatomen im Alkoholteil substituierten Alkylrest mit 1-6 Kohlenstoffatomen, einen gegebenenfalls durch Halogen, eine Nitrogruppe, eine Alkylgruppe mit 1-3 Kohlenstoffatomen, eine Alkoxy- bzw. Alkylmercaptogruppe mit jeweils 1-3 Kohlenstoffatomen substituierten Phenylrest bedeutet, sowie ferner einen Acylrest $R^{11}$ CO- bzw., $R^{12}$ $SO_2$- darstellt, wobei $R^{11}$ bzw. $R^{12}$ Alkylreste mit 1-6 Kohlenstoffatomen oder gegebenenfalls substituierte Phenylreste darstellen, sowie ein

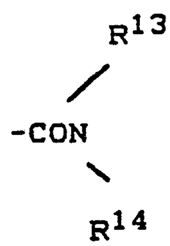 oder 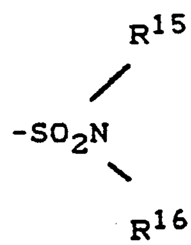 -Rest

sein kann, wobei die Reste $R^{13}$ bis $R^{16}$ Wasserstoff, Alkyl mit 1-6 Kohlenstoffatomen oder einen gegebenenfalls substituierten Phenylrest darstellen,

$R^1$, $R^2$, $R^3$, $R^4$, $R^5$ und $R^6$ für Wasserstoff, eine gegebenenfalls ein- oder mehrfach durch Halogen, insbesondere Chlor oder Fluor, ferner Nitro, Cyano, Hydroxy, Alkoxy bzw. Alkylmercapto mit 1-3 Kohlenstoffatomen, Aryloxy, Arylthio oder eine Estergruppe mit 1-3 Kohlenstoffatomen im Alkoholteil substituierte Alkylgruppe mit 1-6 Kohlenstoffatomen, einen gegebenenfalls durch Halogen, Nitro, Alkyl- bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen, sowie

$R^1$ und $R^2$, $R^3$ und $R^4$, $R^5$ und $R^6$ jeweils mit dem Kohlenstoff, das sie verbindet, einen 3-7 gliedrigen Ring bilden, der gegebenenfalls durch evtl. substituierte Alkylreste mit 1-3 Kohlenstoffatomen oder gegebenenfalls substituierte Arylreste substituiert sein kann, und ferner

$R^2$ mit $R^3$ und/oder $R^4$ mit $R^5$ jeweils mit dem Kohlenstoffatom, an das sie gebunden sind, einen 3-7 gliedrigen Ring bilden, der gegebenenfalls durch gegebenenfalls substutierte Alkylreste mit 1-3 Kohlenstoffatomen oder gegebenenfalls substituierte Arylreste substituiert ist, und

n = 0 oder 1 bedeutet, und wenn n = 1 , $R^5$ und $R^6$ Halogen, Hydroxy, Alkoxy bzw. Alkylmercapto mit 1-3 Kohlenstoffatomen, eine Dialkylaminogruppe mit 1-3 Kohlenstoffatomen in den Alkylresten bedeuten,

dadurch gekennzeichnet, daß man Enamine der Formel II

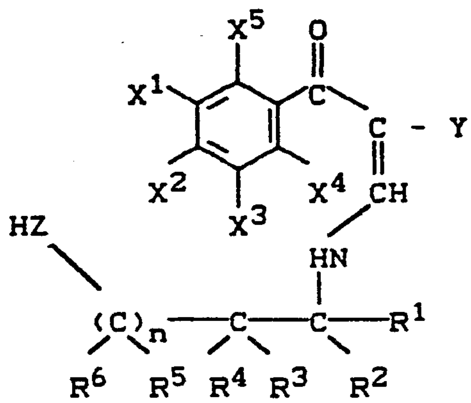

(II)

in der

die Reste $X^1$, $X^2$, $X^3$, $X^4$, $X^5$, $R^1$-$R^6$, Z und Y sowie das Symbol n die oben angegebene Bedeutung haben,

in einer ersten Reaktionsstufe in einem wasserfreien aprotischen Lösungsmittel in Gegenwart von 1 Äquivalent einer Base bei Temperaturen von 80°C bis 180°C zu 4-Chinolon-3-carbonsäurederivaten der Formel III

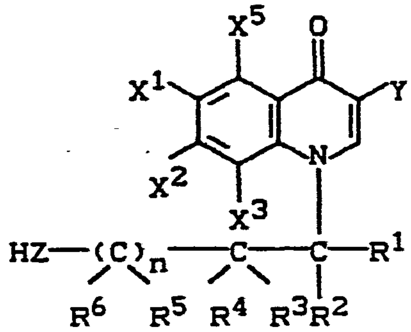

(III)

in der die Reste $X^1$, $X^2$, $X^3$, $X^5$, $R^1$-$R^6$, Z und Y sowie das Symbol n die oben angegebene Bedeutung haben, umsetzt, und in einer zweiten Reaktionsstufe in Gegenwart eines weiteren Äquivalents einer Base die Zweitcyclisierung zu den 1,8-verbrückten 4-Chinolon-3-carbonsäurederivaten der obengenannten Formel I vornimmt, sowie gegebenenfalls die Gruppe Y in an sich bekannter Weise in die Carboxylgruppe und gegebenenfalls diese in ihre Salze überführt.

2. Verfahren nach Anspruch 1, dadurch gekennzeichnet, daß man die Enamine der Formel II mit 2 Äqivalenten einer Base, ohne Zwischenisolierung der Verbindungen III, in einem Eintopfverfahren zu den 1,8-überbrückten 4-Chinolon-3-carbonsäurederivaten I umsetzt.

3. Verfahren nach den Ansprüchen 1 und 2, dadurch gekennzeichnet, daß man die Cyclisierungsreaktionen (II)⟶(III), (III)⟶(I) und (II)⟶(I) bei Temperaturen von 60 bis 300 °C durchführt.

4. Verfahren nach den Ansprüchen 1 und 2, dadurch gekennzeichnet, daß man die Cyclisierungsreaktionen (II)⟶(III), (III)⟶(I) und (II)⟶(I) bei Temperaturen von 80 bis 180 °C durchführt.

5. Verfahren nach den Ansprüchen 1 bis 4, dadurch gekennzeichnet, daß man die Cyclisierungsreaktionen (II)⟶(III), (III)⟶(I) und (II)⟶(I) beim Normaldruck durchführt.

6. Verfahren nach den Ansprüchen 1 bis 4, dadurch gekennzeichnet, daß man die Cyclisierungsreaktionen (II)⟶(III), (III)⟶(I) und (II)⟶(I) bei Drucken von 1 bis 100 bar durchführt.

7. Verfahren nach den Ansprüchen 1 bis 4, dadurch gekennzeichnet, daß man die Cyclisierungsreaktionen (II)⟶(III), (III)⟶(I) und (II)⟶(I) bei Drucken von 1 bis 10 bar durchführt.

8. Verfahren nach den Ansprüchen 1 bis 7, dadurch gekennzeichnet, daß man die Cyclisierungsreaktionen in Dioxan, DMSO, N-Vinylpyrrolidon, Sulfolan, Hexamethylphosphorsäuretrisamid oder DMF oder in Gemischen dieser Lösungsmittel durchführt.

9. Verfahren nach den Ansprüchen 1 bis 8, dadurch gekennzeichnet, daß man die Cyclisierungsreaktonen in Gegenwart von Säurebindern aus der Gruppe bestehend

Kalium-tert.-butanolat, 1,4-Diaza-bicyclo /2,2,2/-octan (DABCO), 1,8-Diaza-bicyclo /5,4,0/undec-7-en (DBU), Butyl-lithium, Lithium-phenyl, Phenyl-magnesiumbromid, Natriummethylat, Natriumethylat, Natriumhydrid, Natrium- und Kaliumcarbonat durchführt.

10. Verwendung von Verbindungen der Formel I

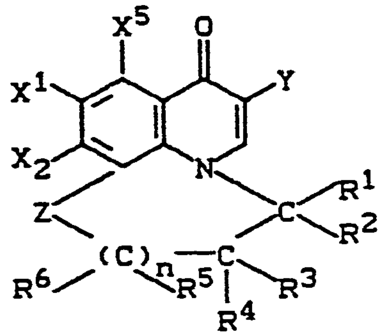

(I)

in der

Y eine Carboxylgruppe, eine Nitrilgruppe, eine Estergruppe-$COOR^7$ oder eine Säureamidgruppe-$CONR^8R^9$ darstellt, wobei $R^7$ für $C_1$-$C_4$-Alkyl steht, $R^8$ und $R^9$ für Wasserstoff oder $C_1$-$C_4$-Alkyl stehen, $R^9$ außerdem gegebenenfalls substituiertes Phenyl sein kann,

$X^1$ für Wasserstoff, Nitro, Alkyl mit 1-3 Kohlenstoffatomen, Halogen, bevorzugt Fluor steht,

$X^2$ Halogen, bevorzugt Fluor oder Chlor, Alkyl mit 1-3 Kohlenstoffatomen, eine Alkylsulfonylgruppe mit bis zu 3 C-Atomen im Alkylrest sowie eine gegebenenfalls im Arylrest substituierte Phenylsulfonylgruppe bedeutet,

$X^5$ Wasserstoff, Halogen oder Methyl sein kann,

Z für Sauerstoff, einen Aminrest $NR^{10}$ steht, wobei $R^{10}$ Wasserstoff, einen gegebenenfalls durch Halogen, Trifluormethyl, Nitro, Cyano, Hydroxy, Alkoxy bzw. Akylmercapto mit 1-3 Kohlenstoffatomen, Aryloxy, Arlythio oder Esterrest mit 1-3 Kohlenstoffatomen im Alkoholteil substituierten Alkylrst mit 1-6 Kohlenstoffatomen, einen gegebenenfalls durch Halogen, eine Nitrogruppe, eine Alkylgruppe mit 1-3 Kohlenstoffatomen, eine Akoxy- bzw. Alkylmercaptogruppe mit jeweils 1-3 Kohlenstoffatomen substituierten Phenylrest bedeutet, sowie ferner einen Acylrest $R^{11}$ CO- bzw., $R^{12}$ $SO_2$- darstellt, wobei $R^{11}$ bzw. $R^{12}$ Alkylreste mit 1-6 Kohlenstoffatomen oder gegebenenfalls substituierte Phenylreste darstellen, sowie ein

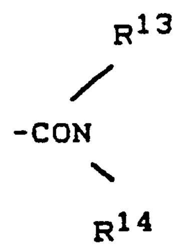 oder 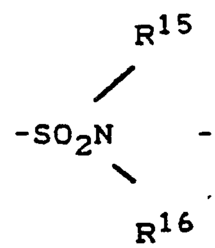 -Rest

sein kann, wobei die Reste $R^{13}$ bis $R^{16}$ Wasserstoff, Alkyl mit 1-6 Kohlenstoffatomen oder einen gegebenenfalls substituierten Phenylrest darstellen,

$R^1$, $R^2$, $R^3$, $R^4$, $R^5$ und $R^6$ für Wasserstoff, eine gegebenenfalls ein- oder mehrfach durch Halogen, insbesondere Chlor oder Fluor, ferner Nitro, Cyano, Hydroxy, Alkoxy bzw. Alkylmercapto mit 1-3 Kohlenstoffatomen, Aryloxy, Arylthio oder eine Estergruppe mit 1-3 Kohlenstoffatomen im Alkoholteil substituierte Alkylgruppe mit 1-6 Kohlenstoffatomen, einen gegebenenfalls durch Halogen, Nitro, Alkyl- bzw. Alkoxy bzw. Alkylmercapto mit jeweils bis zu 3 Kohlenstoffatomen, Hydroxy, Aryloxy, Arylthio, Cyano oder einen Esterrest mit 1-3 Kohlenstoffatomen im Alkoholteil substituierten Phenylrest, Naphthylrest oder heterocyclischen Rest wie z.B. Thiophen-, Furan-, Pyrrol-, Thiazol-, Pyridin- oder Pyrimidin-Rest stehen, sowie

$R^1$ und $R^2$, $R^3$ und $R^4$, $R^5$ und $R^6$ jeweils mit dem Kohlenstoff, das sie verbindet, einen 3-7 gliedrigen Ring bilden, der gegebenenfalls durch evtl. substituierte Alkylreste mit 1-3 Kohlenstoffatomen oder gegebenenfalls substituierte Arylreste substituiert sein kann, und ferner

$R^2$ mit $R^3$ und/oder $R^4$ mit $R^5$ jeweils mit dem Kohlenstoffatom, an das sie gebunden sind, einen 3-7 gliedrigen Ring bilden, der gegebenenfalls durch gegebenenfalls substutierte Alkylreste mit 1-3 Kohlenstoffatomen oder gegebenenfalls substituierte Arylreste substituiert ist, und

$n$ = 0 oder 1 bedeutet, und wenn $n$ = 1, $R^5$ und $R^6$ Halogen, Hydroxy, Alkoxy bzw. Alkylmercapto mit 1 bis 3 Kohlenstoffatomen, eine Dialkylaminogruppe mit 1 bis 3 Kohlenstoffatomen in den Alkylresten bedeuten,

zur Herstellung von Arzneimitteln.

11. Verfahren zur Herstellung der Verbindungen der Formel (II) gemäß Anspruch 1 durch Umsetzung der entsprechenden Enolether der Formel (IV)

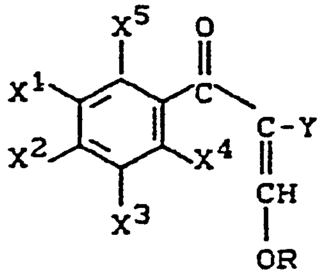

(IV)

mit

R = $CH_3$, $C_2H_5$, $C_3H_7$-n

mit den primären Aminen der Formel (V)

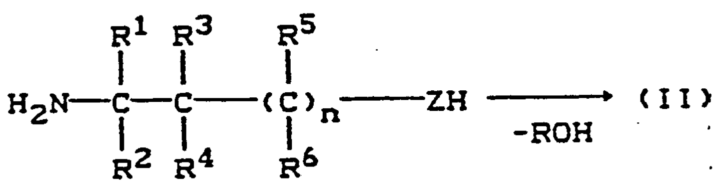


(V)

wobei $X^1$-$X^5$, $R^1$-$R^6$, Z, Y und n die in Anspurch 1 angegebene Bedeutung haben.

Europäisches Patentamt

**EUROPÄISCHER RECHERCHENBERICHT**

0206101
Nummer der Anmeldung

EP 86107908.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 047 005 (DAIICHI SEIYA-KU) * Beispiel 8 * | 6 | C 07 D 498/06<br>C 07 D 471/06<br>C 07 D 215/56<br>C 07 C 101/34<br>C 07 C 121/76<br>A 61 K 31/535<br>A 61 K 31/495 |
| A | * Seiten 5,8-11 * | 1,7-9 | |
| | -- | | |
| X | US - A - 4 499 270 (J.F.GERSTER) * Ansprüche 1-5; Spalte 2, Zeilen 42-45; Spalte 4, Zeilen 24-34 * | 6-9 | |
| | -- | | |
| X | PATENT ABSTRACTS OF JAPAN, unexamined applications, Sektion C, Vol. 7, Nr. 34, 10. Feber 1983<br>THE PATENT OFFICE JAPANESE GOVERNMENT<br>Seite 140 C 150<br>* Kokai-Nr. 57-188 592 (DAIICHI SEIYAKU), siehe Formel II * | 6 | **RECHERCHIERTE SACHGEBIETE (Int. Cl.4)** |
| | -- | | |
| A | PATENT ABSTRACTS OF JAPAN, unexamined applications, Sektion C, Vol. 7, Nr. 53, 3. März 1983<br>THE PATENT OFFICE JAPANESE GOVERNMENT<br>Seite 131 C 154<br>* Kokai-Nr. 57-203 085 (DAIICHI SEIYAKU) * | 6-9 | C 07 D 498/00<br>C 07 D 471/00<br>C 07 D 215/00<br>C 07 C 101/00<br>C 07 C 121/00 |
| | -- | | |
| X | CH - A - 555 339 (SUMITOMO) * Ansprüche I,II * | 5 | |
| | -- | | |

Der vorliegende Recherchenbericht wurde für alle Patentansprüche erstellt.

| Recherchenort | Abschlußdatum der Recherche | Prüfer |
|---|---|---|
| WIEN | 18-09-1986 | JANISCH |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

0206101
Nummer der Anmeldung

-2-
EP 86107908.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 106 013 (KYORIN SEIYAKU)<br>* Ansprüche, Beispiel 14 *<br>-- | 5,7-9 | |
| A | DD - A - 62 321 (G.DOMSCHKE et al.)<br>* Gesamt *<br>-- | 4,10 | |
| P,A | EP - A1 - 0 163 107 (HENKEL)<br>* Anspruch 1 *<br>---- | 4 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 18-09-1986 | JANISCH |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82